# EUROPEAN PATENT SPECIFICATION

(11) **EP 2 608 847 B1**
(45) Date of publication and mention of the grant of the patent: **21.03.2018**
(21) Application number: 11755105.1
(22) Date of filing: 25.08.2011
(51) Int. Cl.: A61P 11/10, A61P 11/12, A61P 11/14, A61K 31/047, A61K 31/085, A61K 31/09, A61K 31/122, A61K 31/137, A61K 31/185, A61K 31/197, A61K 31/198, A61K 31/223, A61K 31/235, A61K 31/265, A61K 31/357, A61K 31/522, A61K 9/16, A61K 45/06

(54) **THEOBROMINE IN COMBINATION WITH AN EXPECTORANT OR A MUCOLYTIC FOR USE IN THERAPY**
THEOBROMIN IN KOMBINATION MIT EINEM HUSTEN- ODER SCHLEIMLÖSENDEN MITTEL FÜR THERAPIEZWECKE
THÉOBROMINE COMBINÉE À UN EXPECTORANT OU À UN MUCOLYTIQUE ET SON UTILISATION EN THÉRAPIE

(30) Priority: 27.08.2010 GB 201014391
(43) Date of publication of application: 03.07.2013
(73) Proprietor: InFirst Healthcare Limited, London WC2R 1BH (GB)
(72) Inventor: BANNISTER, Robin, Mark, London Greater London EC2Y 8AD (GB); BREW, John, London Greater London EC2Y 8AD (GB)
(74) Representative: Schlich, George
(86) International application number: PCT/GB2011/051610
(87) International publication number: WO 2012/025761

(56) References cited:
- EP-A1- 2 050 435
- WO-A1-00/00212
- WO-A1-2010/146394
- WO-A1-2011/146031
- WO-A1-2013/004999
- GB-A- 2 424 185
- US-A1- 2005 220 897
- US-A1- 2006 148 837
- US-A1- 2008 085 312
- US-A1- 2008 176 955
- DATABASE WPI Week 200343 Thomson Scientific, London, GB; AN 2003-451953 XP002665279, -& JP 2003 055258 A (ROHTO SEIYAKU KK) 26 February 2003 (2003-02-26)
- DATABASE WPI Week 200308 Thomson Scientific, London, GB; AN 2003-078587 XP002665280, -& JP 2002 193839 A (MEIJI SEIKA KAISHA LTD) 10 July 2002 (2002-07-10)
- USMANI OMAR S ET AL: "Theobromine inhibits sensory nerve activation and cough", FASEB JOURNAL, vol. 19, no. 2, 1 February 2005 (2005-02-01), pages 231-233, XP002609390, FED. OF AMERICAN SOC. FOR EXPERIMENTAL BIOLOGY, US ISSN: 0892-6638, DOI: 10.1096/FJ.04-1990FJE [retrieved on 2004-11-17] cited in the application
- Mostafa Yakoot ET AL: "Clinical Efficacy of farcosolvin syrup (ambroxol-theophylline-guaiphenesin mixture) in the treatment of acute exacerbation of chronic bronchitis.", International Journal of Chronic Obstructive Pulmonary Disease, vol. 5 20 July 2010 (2010-07-20), pages 251-256, XP055292970, Retrieved from the Internet: URL:http://www.ncbi.nlm.nih.gov/pmc/articl es/PMC2921693/pdf/copd-5-251.pdf

## Description

### Field of the Invention

This invention relates to a drug combination, its composition and its use in therapy.

### Background of the Invention

Cough is a protective reflex. Persistent cough can be distressing. Over-the-counter remedies are available but their effectiveness is doubtful.

WO98/42322 discloses the use of theobromine for the treatment of cough, to be given orally.

Usmeni et al., FASEB J. express article 10.1096, discloses that theobromine inhibits sensory nerve action and cough. Data are provided, showing effects following oral dosing in citric-acid induced cough in the guinea pig, and in the capsaicin cough challenge in humans, and following bathing of isolated guinea pig vagus nerve preparations.

Expectorants and mucolytic agents dissolve thick mucus and relieve respiratory difficulties. Expectorants reduce the viscosity of bronchial secretions thus increasing mucus flow that can then be removed by coughing. Mucolytic agents also thin mucus, but do so by breaking down the chemical structure of mucus molecules.

Expectorants and mucolytics thin the mucus protective barrier, which as a result maximizes the penetration of pro-tussive agents (such as citric acid, dust, allergens etc) into the lung and enhances the pro-tussive effect.

### Summary of the Invention

Given that a mucolytic or an expectorant is potentially pro-tussive, it was surprising to find that a mucolytic/expectorant and theobromine had synergy. Based on what is known about expectorants/mucolytics, it was expected that an expectorant/mucolytic would decrease the efficacy of theobromine.

The invention is therefore based at least in part on data showing synergistic anti-tussive effects for theobromine combined with guaifenesin in a citric acid-induced cough model. When guaifenesin is combined with theobromine, the effects are surprisingly potent and greater than the sum of the individual drugs, revealing that the combination has a substantially improved effect. Consequently, a considerably reduced dose of both drugs can be given for an equivalent effect for each individual drug, so reducing side-effects and drug burden.

Therefore, the present invention is an agent consisting of theobromine and guaifenesin as sole active agents for use as a combined preparation in cough therapy.

### Description of the Figures

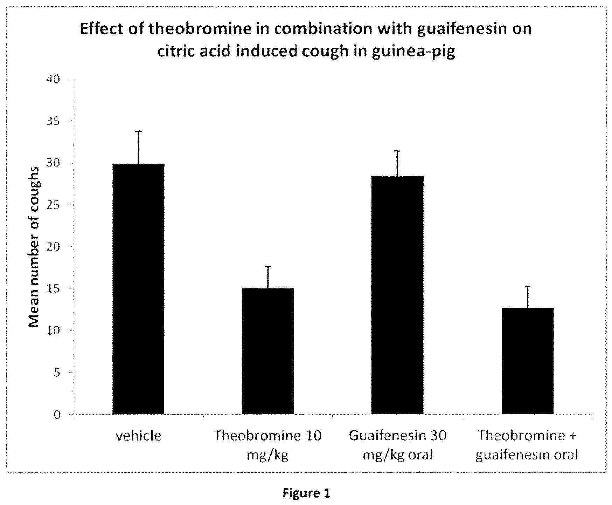

Figure 1 shows the total number of coughs in guinea-pigs during a 10 minute exposure and 5 minute recovery period to citric acid (1M), following pre-treatment with either vehicle (p.o.), theobromine (10 mg kg⁻¹, i.p), guaifenesin (30 mg/kg, p.o.) or theobromine (10 mg/kg, p.o.) combined with guaifenesin (30 mg/kg. p.o.). Each column represents the mean and the vertical bars the s.e. mean. Changes in total cough number in the presence of theobromine, guaifenesin and theobromine in combination with guaifenesin were compared to vehicle dosed animals using ANOVA followed by Dunnetts test to compare the means. **P<0.01, *P<0.05.

### Description of the Invention

Any suitable form of theobromine can be chosen. These include salts. The additional agent (expectorant or mucolytic which is guaifenesin) may be used in an amount that is already known for its use, although combination according to this invention means that a reduced dose may be effective. A combination according to the invention may be provided in a single formulation or in separate formulations, for combined, simultaneous or sequential administration. Also disclosed are expectorants such as ambroxol, ammonium bicarbonate, ammonium carbonate, bromhexine, calcium iodide, carbocysteine, guaiacol, guaicacol benzoate, guaiacol carbonate, guaiacol phosphate, guaithylline, hydriodic acid, iodinated glycerol, potassium guaiacolsulfonate, potassium iodide, sodium citrate, sodium iodide, storax terebene, terpin, trifolium, Althea root, Antimony pentasulfide, Creosote, Ipecacuanha (Syrup of ipecac), levoverbenone, Senega and Tyloxapol. Preferably, the expectorant is guaifenesin. Also disclosed are mucolytics such as acetylcysteine, bromhexine, carbocysteine, domiodol, erdostine, letostine, lysozyme, mecysteine hydrochloride, mesna, sobrerol, stepronin, tiopronin, tyloxapol, ambroxol, ammonium chloride, Dornase alfa, Eprazinone, Erdosteine, Letosteine and Neltenexine. Also disclosed is an agent which comprises theobromine, an expectorant and a mucolytic. Also disclosed is an agent which consists essentially of theobromine and an expectorant, or consists essentially of theobromine and a mucolytic, or consists essentially of theobromine, an expectorant and a mucolytic. Also disclosed is an agent which consists of theobromine and an expectorant as active agents, or consists of theobromine and a mucolytic as active agents, or consists of theobromine, an expectorant and a mucolytic as active agents.

Any suitable form of theobromine can be chosen. As theobromine is present in cocoa, the theobromine may be added in the form of cocoa or chocolate (provided the amount of theobromine is in a high enough concentration to provide a therapeutic effect in the combination). If the theobromine is added in the form of cocoa and the medicament combination is in the form of a liquid, then there is the added benefit that the medicament is pleasant tasting.

The compounds of the invention are preferably as combinations to be administered orally, for example as tables, troches, lozenges, aqueous or orally suspensions, dispersible powders or granules. Preferred pharmaceutical compositions of the invention are tablets and capsules. Liquid dispersions for oral administration may be syrups, emulsions and suspensions. More preferably, the pharmaceutical composition of the combination is a pressed tablet or capsule with conventional excipients, examples of which are given below.

Compositions of the combination intended for oral use may be prepared according to any method known to the art for the manufacture of pharmaceutical compositions, and such compositions may contain one or more agents selected from the group consisting of sweetening agents, flavouring agents, colouring agents and preserving agents in order to provide pharmaceutically elegant and palatable preparations. Tablets contain the combined active ingredients in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example corn starch or alginic acid; binding agents, for example starch gelatin, acacia, microcrystalline cellulose or polyvinyl pyrrolidone; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Aqueous suspensions contain the combined active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl pyrrolidone, gum tragacanth and gum acacia; dispersing or wetting agents may be a naturally occurring phosphatide, for example lecithin, or condensation products of an alkylene oxide with fatty acids, for example polyoxyethylene stearate, or condensation products of ethylene oxide with long-chain aliphatic alcohols, for example heptadecaethyleneoxycetanol, or condensation products of ethylene oxide with partial esters derived from fatty acids, for example polyoxyethylene sorbitan monooleate. The aqueous suspensions may also contain one or more preservatives, for example ethyl or n-propyl p-hydroxybenzoate, one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, polyoxyethylene hydrogenated castor oil, fatty acids such as oleic acid, or in a mineral oil such as liquid paraffin or in other surfactants or detergents. The oily suspensions may contain a thickening agent, for example beeswax, hard paraffin or cetyl alcohol. Sweetening agents, such as those set forth above, and flavouring agents may be added to provide a palatable oral preparation. These compositions may be preserved by the addition of an antioxidant such as ascorbic acid.

Dispersible powders and granules suitable for preparation of an aqueous suspension by the addition of water provide the combined active ingredients in admixture with a dispersing or wetting agent, suspending agent and one or more preservatives. Suitable sweetening, flavouring and colouring agents may also be present.

The combined pharmaceutical compositions of the invention may also be in the form of oil-in-water emulsions. The oily phase may be a vegetable oil, for example olive oil or arachis oil, or a mineral oil, for example liquid paraffin, or mixtures of these. Suitable emulsifying agents may be naturally occurring gums, for example gum acacia or gum tragacanth, naturally occurring phosphatides, for example soya bean, lecithin, and esters or partial esters derived from fatty acids and hexitol anhydrides, for example sorbitan monooleate and condensation products of the said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative, flavouring and colouring agents.

Suspensions and emulsions may contain a carrier, for example a natural gum, agar, sodium alginate, pectin, methylcellulose, carboxymethylcellulose, or polyvinyl alcohol.

In a preferred embodiment, theobromine in combination with an expectorant and/or a mucolytic which is guaifenesin is to be administered via the oral route. Combined compositions according to the invention may be produced using conventional formulation techniques. In particular, spray-drying may be used to produce microparticles comprising the active agent dispersed or suspended within a material that provides the controlled release properties.

The process of milling, for example jet milling, may also be used to formulate the therapeutic composition. The manufacture of fine particles by milling can be achieved using conventional techniques. The term "milling" is used herein to refer to any mechanical process which applies sufficient force to the particles of active material to break or grind the particles down into fine particles. Various milling devices and conditions are suitable for use in the production of the compositions of the invention. The selection of appropriate milling conditions, for example, intensity of milling and duration, to provide the required degree of force, will be within the ability of the skilled person. Ball milling is a preferred method. Alternatively, a high pressure homogeniser may be used, in which a fluid containing the particles is forced through a valve at high pressure, producing conditions of high shear and turbulence. Shear forces on the particles, impacts between the particles and machine surfaces or other particles, and cavitation due to acceleration of the fluid, may all contribute to the fracture of the particles. Suitable homogenisers include the EmulsiFlex high pressure homogeniser, the Niro Soavi high pressure homogeniser and the Microfluidics Microfluidiser. The milling process can be used to provide the microparticles with mass median aerodynamic diameters as specified above. If hygroscopic, the active agent may be milled with a hydrophobic material, as stated above.

If it is required, the microparticles produced by the milling step can then be formulated with an additional excipient. This may be achieved by a spray-drying process, e.g. co-spray-drying. In this embodiment, the particles are suspended in a solvent and co-spray-dried with a solution or suspension of the additional excipient. Preferred additional excipients include polysaccharides. Additional pharmaceutically effective excipients may also be used.

Compositions of the combination intended for inhaled, topical, intranasal, sublingual, intravenous, rectal and vaginal use may be prepared according to any method known to the art for the manufacture of pharmaceutical compositions.

Any suitable pharmaceutically effective drug which is used for the treatment of a respiratory disease is also disclosed. For example, β₂-agonists, e.g. salbutamol, salmeterol and formoterol, may be formulated for co-administration. Additional anti-muscarinic compounds may also be co-administered. For example, ipratropium (e.g. ipratropium bromide) or tiotropium may be administered.

Additional therapeutic agents, including steroids, may also be co-administered. Examples of suitable steroids include beclomethasone, dipropionate and fluticasone. Other suitable therapeutic agents suitable for co-administration include mucolytics, matrix metalloproteinase inhibitors, leukotrienes, antibiotics, anti-infective agents, antineoplastics, peptides, antitussives, nicotine, PDE4 inhibitors, elastase inhibitors and sodium cromoglycate.

Therapy according to the invention may be conducted in generally known manner, depending on various factors, such as the sex, age or condition of the patient, and the existence or otherwise of one or more concomitant therapies. The patient population may be important, for example, in the treatment of patients with liver disease.

The amounts of theobromine in the combination in one unit dose may be from 1 mg to 3000 mg. Preferably, the unit dose is from 50 mg to 1500 mg. More preferably, the unit dose is from 400 mg to 1000 mg to be dosed one to four times daily.

In a preferred embodiment, theobromine is to be dosed in an amount from 1-1000 mg/kg, preferably from 5-50 mg/kg.

In a preferred embodiment, the expectorant and/or mucolytic which is guaifenesin is to be dosed in an amount from 1-1000 mg/kg, preferably from 1-100 mg/kg.

The following study illustrates the invention.

### Study

### Test Doses

Group 1 - vehicle control
Group 2 - theobromine (10 mg/kg, p.o.)
Group 3 - guaifenesin (30 mg/kg, p.o.)
Group 4 - theobromine (10 mg/kg, p.o.) + guaifenesin (30 mg/kg, p.o.)

### Methods

### Animals

Male Dunkin Hartley guinea pigs (400-510 g, supplied by Harlan UK Ltd) were used throughout the study.

### Protocol

Twenty four guinea pigs were randomly allocated to one of the three treatment groups (see Table 1) according to the blinding code. The blinding code was not revealed to the investigator until coughs from all of the animals had been tallied.

Guinea pigs were dosed via oral gavage (dose volume 2 mL/kg) with theobromine administered 0.5 hours prior to citric acid exposure. Guaifenesin and vehicle were administered 60 min prior to citric acid exposure.

Individual guinea pigs were placed in an exposure chamber with an airflow of 2 L/min at t -10 min prior to citric acid exposure to allow acclimatisation. At t = 0 min, cough responses were induced by exposure to citric acid aerosol (1 M) generated by an ultrasonic nebuliser at a nebulisation rate of 0.6 mL/min for 10 min.

Coughs were counted throughout the 10 min citric acid exposure and for a further 5 min post exposure.

**Table 1: Treatment groups for study 376 (n=6 guinea pigs per group).**

| **Vehicle Group** | **Treatment group 1** | **Treatment group 2** | **Treatment group 3** |
|---|---|---|---|
| | theobromine (10 mg/kg,p.o.), 0.5h pretreatment | guaifenesin (30 mg/kg, p.o.), 1h pretreatment | Theobromine (10 mg/kg, p.o.) + guaifenesin, (16 mg/kg, p.o.), 0.5h and 1h pretreatment respectively |

### Results

The mean number of citric acid induced cough responses recorded in the vehicle treated guinea pigs was 30 ± 4 coughs. Pre-treatment with theobromine (10 mg/kg, p.o.) caused a significant (P<0.05) reduction to the number of citric acid induced coughs (15 ± 3). In contrast though, guaifenesin (30 mg/kg, p.o.) had no significant effect on the total number of coughs (28 ± 3) evoked by citric acid exposure. In combination with guaifenesin (30 mg/kg, p.o.), the inhibitory effect of theobromine on the citric acid-induced tussive activity, showed a trend to be potentiated in respect to total number of coughs observed (13 ± 3 c.f. 15 ± 3). This synergy is shown in Figure 1.

## Claims

1. An agent consisting of theobromine and guaifenesin as active agents for use as a combined preparation in cough therapy.

2. An agent for use according to claim 1, wherein the theobromine is for administration in a dose of from 1 to 3000 mg.

3. An agent for use according to claim 2, wherein the theobromine is for administration in a dose of from 50 to 1500 mg.

4. An agent for use according to claim 3, wherein the theobromine is for administration in a dose of from 400 to 1000 mg.

5. An agent for use according to any one of claims 1-4, for oral administration.

6. An agent for use according to any one of claims 1-5, for oral administration as a tablet, capsule, troche, lozenge, dispersible powder, granule, suspension, syrup or emulsion.

7. A pharmaceutical composition for oral administration comprising an agent as defined in any one of claims 1-4, one or more excipients and optionally one or more of sweetening agents, flavouring agents, colouring agents and/or preserving agents, for use in cough therapy.

8. A pharmaceutical composition for use according to claim 7, for oral administration as a tablet, capsule, troche, lozenge, dispersible powder, granule, suspension, syrup or emulsion.

## Patentansprüche

1. Mittel, bestehend aus Theobromin und Guaifenesin als Wirkstoffen, zur Verwendung als Kombinationspräparat bei der Hustentherapie.

2. Mittel zur Verwendung nach Anspruch 1, wobei das Theobromin zur Verabreichung in einer Dosis von 1 bis 3000 mg vorgesehen ist.

3. Mittel zur Verwendung nach Anspruch 2, wobei das Theobromin zur Verabreichung in einer Dosis von 50 bis 1500 mg vorgesehen ist.

4. Mittel zur Verwendung nach Anspruch 3, wobei das Theobromin zur Verabreichung in einer Dosis von 400 bis 1000 mg vorgesehen ist.

5. Mittel zur Verwendung nach einem der Ansprüche 1-4 zur oralen Verabreichung.

6. Mittel zur Verwendung nach einem der Ansprüche 1-5 zur oralen Verabreichung als Tablette, Kapsel, Pastille, Lutschtablette, dispergierbares Pulver, Granulat, Suspension, Sirup oder Emulsion.

7. Pharmazeutische Zusammensetzung zur oralen Verabreichung, umfassend ein Mittel entsprechend der Definition nach einem der Ansprüche 1-4, ein oder mehrere Hilfsstoffe und gegebenenfalls eines oder mehrere von Süßungsmitteln, Aromastoffen, Farbstoffen und/oder Konservierungsstoffen zur Verwendung bei der Hustentherapie.

8. Pharmazeutische Zusammensetzung zur Verwendung nach Anspruch 7 zur oralen Verabreichung als Tablette, Kapsel, Pastille, Lutschtablette, dispergierbares Pulver, Granulat, Suspension, Sirup oder Emulsion.

## Revendications

1. Agent constitué de théobromine et de guaifénésine comme agents actifs pour utilisation en préparation combinée dans le traitement de la toux.

2. Agent pour utilisation selon la revendication 1, dans lequel la théobromine doit être administrée en dose de 1 à 3000 mg.

3. Agent pour utilisation selon la revendication 2, dans lequel la théobromine doit être administrée en dose de 50 à 1500 mg.

4. Agent pour utilisation selon la revendication 3, dans lequel la théobromine doit être administrée en dose de 400 à 1000 mg.

5. Agent pour utilisation selon l'une quelconque des revendications 1-4, pour administration orale.

6. Agent pour utilisation selon l'une quelconque des revendications 1-5, pour administration orale sous forme de comprimé, capsule, trochisque, pastille, poudre dispersable, granule, suspension, sirop ou émulsion.

7. Composition pharmaceutique pour administration orale, comprenant un agent tel que défini dans l'une quelconque des revendications 1-4, un ou plusieurs excipients et optionnellement un ou plusieurs édulcorants, aromatisants, colorants et/ou conservateurs, pour utilisation dans le traitement de la toux.

8. Composition pharmaceutique pour utilisation selon la revendication 7, pour administration orale sous forme de comprimé, capsule, trochisque, pastille, poudre dispersable, granule, suspension, sirop ou émulsion.
